# EUROPEAN PATENT APPLICATION

(11) **EP 4 629 261 A1**
(43) Date of publication of application: **08.10.2025**
(21) Application number: 25167484.2
(22) Date of filing: 31.03.2025
(51) Int. Cl.: G16H 40/67

(54) **UNIVERSAL DOCKING STATION WITH LOCATION CONTEXT**

(30) Priority: 03.04.2024 US 202463573894 P
(71) Applicant: Drägerwerk AG & Co. KGaA, 23558 Lübeck (DE)
(72) Inventor: RISHER-KELLY, Clifford M, Wells, 04090 (US)
(74) Representative: Haseltine Lake Kempner LLP

(57) **Abstract**

A docking station is disclosed that is adapted to append location information into the sensor signal data stream of intelligent sensors connected to docking station ports and enable that location information for each sensor to be communicated to a hospital network even when a multi-parameter patient monitor is not present. The docking station may include a graphical user interface that displays sensor status data, generates sensor-related alarms, and enables users to silence the alarms via the graphical user interface.

## Description

### FIELD

The subject matter of the present disclosure relates generally to devices for updating data in medical monitoring or therapeutic systems. More specifically, described herein is a docking station which informs a sensor of the sensor's location and allows the sensor to store and connect and communicate its location to other devices including a medical monitoring device and other output devices.

### BACKGROUND

A conventional docking station associates its connected module(s) with a monitor that is connected to the docking station. A sensor is a type of module. In a medical setting, a patient may be monitored by one or more patient physiological sensors that provide parameters and values used for measurement and/or monitoring the patient, for example. The sensors are connected to a medical monitoring device via ports on the monitor. For some patients, additional sensors are required which may exceed the number of ports on the monitor. In this case, additional ports are provided by the docking station. It has been standard practice to add one or more sensors to the medical monitoring device through the use of a docking station. Data from the physiological sensors are reported to the medical monitoring device, via the docking station. The medical monitoring device may receive and display the data from the sensors, via the docking station. However, when the medical monitoring device is removed (e.g., disconnected from the docking station), the sensors (and thus the parameters of the sensors) are no longer connected to the medical monitoring device and are no longer connected to a database associated with the patient, and thus can no longer provide data regarding the patient.

It is with respect to these and other considerations that the various aspects and embodiments of the present disclosure are presented.

### SUMMARY

Systems and methods are provided that connect a sensor to a docking station instead of to a monitor such as a medical monitoring device. The docking station informs the sensor of a location of the sensor. The sensor stores the location and communicates the location to the medical monitoring device and/or other output devices such as patient information output devices that aggregate or display patient information.

Systems and methods are provided that provide a docking station that has multiple ports and a user interface. Each port is configured to receive a sensor and is associated with a location. The docking station connects sensors to a network without being connected to a medical monitoring device. The user interface displays a status of at least one sensor that is connected to the docking station.

This summary is provided to introduce a selection of concepts in a simplified form that are further described below in the detailed description. This summary is not intended to identify key features or essential features of the claimed subject matter, nor is it intended to be used to limit the scope of the claimed subject matter.

Aspect 1: A method comprising:
a. providing a first docking station having a power source, a processor, a mount configured to removably receive a multi-parameter patient monitor, at least one sensor port configured to mechanically and electrically connect to an intelligent physiological sensor, a docking station communications interface configured to communicate with a hospital network, and a docking station I/O interface configured to communicate with the intelligent physiological sensor connected to each of the at least one sensor port and the multi-parameter patient monitor connected to the mount, the hospital network being adapted to store location data for the first docking station, the intelligent physiological sensor being adapted to generate a sensor data signal that includes sensor parameter data;
b. associating the first docking station with a first location on the hospital network;
c. establishing an electrical connection between the docking station I/O interface and a sensor data signal for the intelligent physiological sensor when the intelligent physiological sensor is connected to the at least one sensor port; and
d. appending the sensor parameter data using the docking station I/O interface to include a sensor location parameter that associates the intelligent physiological sensor with the first location.

Aspect 2: The method of aspect 1, further comprising:
e. transmitting the appended sensor parameter data from the first docking station through the communications interface to at least one selected from the group of the multi-parameter patient monitor connected to the mount and the hospital network; and
f. using the appended sensor data to associate the intelligent physiological sensor with the first location on the hospital network.

Aspect 3: The method of any of aspects 1-2, wherein step e comprises transmitting the appended sensor parameter data from the first docking station through the communications interface to the hospital network.

Aspect 4: The method of aspect f, wherein the first docking station is adapted to perform step e regardless of whether the multi-parameter patient monitor is connected to the mount.

Aspect. 5: The method of any of aspects 1-4, further comprising:
g. terminating the connection between the docking station I/O interface after steps c and d have been completed.

Aspect 6: The method of any of aspects 1-5, wherein the intelligent physiological sensor is one selected from the group of an intelligent patient front end (IPFE) sensor and a service-oriented device connectivity (SDC) compliant sensor.

Aspect 7: The method of any of aspects 1-6, wherein the hospital network is a service-oriented device connectivity (SDC) network.

Aspect 8: The method of any of aspects 1-7, wherein the first docking station further comprises a graphical user interface and the method further comprises:
h. displaying on the graphical user interface status information for each intelligent physiological sensor electrically connected to the at least one sensor port.

Aspect 9: The method of aspect 6, wherein the status information comprises at least one selected from the group of a sensor type, a sensor status, a status description, and an alarm status.

Aspect 10: The method of any of aspects 6-9, further comprising:
i. generating at least one selected from the group of an audio alarm and a visual alarm through the graphical user interface when the alarm status of at least one of the intelligent physiological sensors electrically connected to the at least one sensor port is active.

Aspect 11: The method of aspect 7, further comprising:
j. enabling a user to silence the alarm generated by step i using the graphical user interface.

Aspect 12: The method of any of aspects 1-11, further comprising:
k. calibrating at least one of the intelligent physiological sensors;
wherein the first docking station is adapted to perform step (k) even if the multi-parameter patient monitor is not connected to the mount.

Aspect 13: The method of any of aspects 1-12, further comprising:
I. warming at least one of the intelligent physiological sensors;
wherein the first docking station is adapted to perform step (I) even if the multi-parameter patient monitor is not connected to the mount.

Aspect 14: The method of any of aspects 1-13, further comprising:
m. disconnecting the intelligent physiological sensor from the first docking station;
n. automatically associating the intelligent physiological sensor with the first location when step (m) is performed;
o. mechanically and electrically connecting the intelligent physiological sensor to a second docking station, the second docking station being associated with a second location on the hospital network; and
p. automatically associating the intelligent physiological sensor with the second location when step (o) is performed.

Aspect: 15: A docking station comprising:
a power source;
a processor;
a mount configured to removably receive a multi-parameter patient monitor;
at least one sensor port configured to mechanically and electrically connect to an intelligent physiological sensor;
a docking station communications interface configured to communicate with a hospital network and to associate a first location with the docking station; and
a docking station I/O interface configured to communicate with the intelligent physiological sensor connected to each of the at least one sensor port and the multi-parameter patient monitor connected to the mount,
wherein the docking station I/O interface is further configured to append the first location to a sensor data signal of the intelligent physiological sensor when the intelligent physiological sensor is electrically connected to the at least one sensor port.

Aspect 16: The docking station of aspect 10, wherein the docking station communications interface is adapted to transmit the sensor data signal to the hospital network even if the multi-parameter patient monitor is not received in the mount.

Aspect 17: The docking station of any of aspects 10-16, wherein the docking station I/O interface is configured to terminate a connection with the intelligent physiological sensor after the first location is appended to the sensor data signal.

Aspect 18: The docking station of any of aspects 10-17, wherein the at least one sensor port comprises at least one intelligent patient front end (IPFE) port.

Aspect 19: The docking station of any of aspects 10-18, wherein the at least one sensor port comprises at least one service-oriented device connectivity (SDC) port.

Aspect 20: The docking station of any of aspects 10-19, further comprising a graphical user interface adapted to provide status information for each intelligent physiological sensor electrically connected to the at least one sensor port.

Aspect 21: The docking station of aspect 14, wherein the status information comprises at least one selected from the group of a sensor type, a sensor status, a status description, and an alarm status.

Aspect 22: The docking station of any of aspects 14-21, wherein the graphical user interface is adapted to generate at least one selected from the group of an audio alarm and a visual alarm through the graphical user interface when the alarm status of at least one of the intelligent physiological sensors electrically connected to the at least one sensor port is active.

Aspect 20: The docking station of any of aspects 14-22, wherein the graphical user interface is adapted to enable a user to silence the alarm generated by the graphical user interface.

### BRIEF DESCRIPTION OF THE DRAWINGS

The foregoing summary, as well as the following detailed description of illustrative embodiments, is better understood when read in conjunction with the appended drawings. Other aspects, features, and advantages of described embodiments will become more fully apparent from the following detailed description, the appended claims, and the accompanying drawings in which like reference numerals identify similar or identical elements. For the purpose of illustrating the embodiments, there is shown in the drawings example constructions of the embodiments; however, the embodiments are not limited to the specific methods and instrumentalities disclosed. In the drawings:
FIG. 1 shows a block diagram of an exemplary environment for providing location context with a docking station in accordance with described embodiments;
FIG. 2 shows a schematic diagram of a docking station in accordance with described embodiments;
FIG. 3 shows a schematic diagram of aspects of the environment of FIG. 1 with additional detail;
FIG. 4 shows another schematic diagram of aspects of the environment of FIG. 1 with additional detail;
FIG. 5 shows another schematic diagram of aspects of the environment of FIG. 1 with additional detail;
FIG. 6 shows an example of an intelligent patient front end (IPFE) and service-oriented device connectivity (SDC) status display of a docking station in accordance with described embodiments;
FIG. 7 is an operational flow of an implementation of a method for providing location context with a docking station in accordance with described embodiments;
FIG. 8 is an operational flow of another implementation of a method for providing location context with a docking station in accordance with described embodiments;
FIG. 9 is an operational flow of another implementation of a method for providing location context with a docking station in accordance with described embodiments;
FIG. 10 is an operational flow of another implementation of a method for providing location context with a docking station in accordance with described embodiments; and
FIG. 11 shows an exemplary computing environment in which example embodiments and aspects may be implemented.

### DETAILED DESCRIPTION

The ensuing detailed description provides example embodiments only, and is not intended to limit the scope, applicability, or configuration of the claims. Rather, the ensuing detailed description provides those skilled in the art with an enabling description for implementing the described embodiments. Various changes might be made in the function and arrangement of described elements without departing from the spirit and scope of the appended claims.

Directional terms may be used in this specification and claims to describe portions of the present invention (e.g., upper, lower, left, right, etc.). These directional terms are merely intended to assist in describing the embodiments and are not intended to limit the scope of the claims. In addition, reference numerals that are introduced in the specification in association with a drawing figure may be repeated in one or more subsequent figures without additional description in the specification in order to provide context for other features.

Reference herein to "one embodiment" or "an embodiment" means that a particular feature, structure, or characteristic described in connection with the embodiment can be included in at least one embodiment. The appearances of the phrase "in one embodiment" in various places in the specification are not necessarily all referring to the same embodiment, nor are separate or alternative embodiments necessarily mutually exclusive of other embodiments. The same applies to the term "implementation."

As used in this application, the word "exemplary" is used herein to mean serving as an example, instance, or illustration. Any aspect or design described herein as "exemplary" is not necessarily to be construed as preferred or advantageous over other aspects or designs. Rather, use of the word exemplary is intended to present concepts in a concrete fashion.

As used in this application, the term "multi-parameter patient monitor" refers to a device that is capable of receiving and displaying patient physiological data, such as heart rate, blood oxygen concentration, respiration, and an electrocardiogram. A multi-parameter patient monitor often includes alarm functionality and will include at least one port that is capable of connecting a sensor that is capable of sensing a particular type of patient physiological data. Some multi-parameter patient monitors include an interface that enables it to be docked with a docking station that may provide additional sensor ports, an additional power source, and additional communication interfaces (such as a wired connection to a hospital network).

One or more aspects of the embodiments may be implemented as processing blocks in a software program, including possible implementation as a digital signal processor, microcontroller, or general-purpose computer; however, described embodiments are not so limited. As would be apparent to one skilled in the art, various functions of software might also be implemented as processes of circuits. Such circuits might be employed in, for example, a single integrated circuit, a multi-chip module, a single card, or a multi-card circuit pack.

Described embodiments might also be embodied in the form of methods and apparatuses for practicing those methods. Described embodiments might also be embodied in the form of program code embodied in non-transitory tangible media, such as magnetic recording media, optical recording media, solid state memory, floppy diskettes, CD-ROMs, hard drives, or any other non-transitory machine-readable storage medium, wherein, when the program code is loaded into and executed by a machine, such as a computer, the machine becomes an apparatus for practicing described embodiments. Described embodiments might also be embodied in the form of program code, for example, whether stored in a non-transitory machine-readable storage medium, loaded into and/or executed by a machine, or transmitted over some transmission medium or carrier, such as over electrical wiring or cabling, through fiber optics, or via electromagnetic radiation, wherein, when the program code is loaded into and executed by a machine, such as a computer, the machine becomes an apparatus for practicing the described embodiments. When implemented on a general-purpose processor, the program code segments combine with the processor to provide a unique device that operates analogously to specific logic circuits. Described embodiments might also be embodied in the form of a bitstream or other sequence of signal values electrically, optically, or wirelessly transmitted through a medium, stored magnetic-field variations in a magnetic recording medium, etc., generated using a method and/or an apparatus of the described embodiments.

It should be understood that the steps of the methods set forth herein are not necessarily required to be performed in the order described, and the order of the steps of such methods should be understood to be presented as examples. Likewise, additional steps might be included in such methods, and certain steps might be omitted or combined, in methods consistent with various described embodiments.

As used herein in reference to an element and a standard, the term "compatible" means that the element communicates with other elements in a manner wholly or partially specified by the standard and would be recognized by other elements as sufficiently capable of communicating with the other elements in the manner specified by the standard. The compatible element does not need to operate internally in a manner specified by the standard. Unless explicitly stated otherwise, each numerical value and range should be interpreted as being approximate as if the word "about" or "approximately" preceded the value of the value or range.

Also for purposes of this description, the terms "couple," "coupling," "coupled," "connect," "connecting," or "connected" refer to any manner known in the art or later developed in which energy is allowed to be transferred between two or more elements, and the interposition of one or more additional elements is contemplated, although not required. Conversely, the terms "directly coupled," "directly connected," etc., imply the absence of such additional elements. Signals and corresponding nodes or ports might be referred to by the same name and are interchangeable for purposes here.

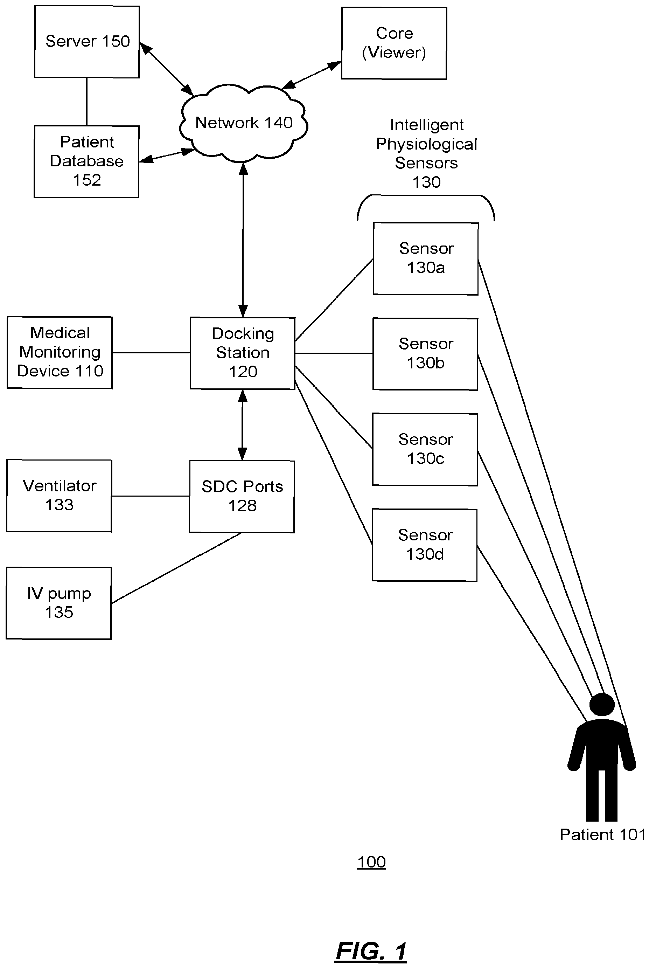

FIG. 1 shows a block diagram of an exemplary environment 100 for providing location context with a docking station in accordance with described embodiments. No aspect of FIG. 1 is intended to be limiting in any sense, for numerous variants are contemplated as well. In some embodiments, a specific location context may correlate to a specific patient context. In some embodiments, patient context may be linked to a specific set of ports on an intelligent patient monitor mount.

The environment 100 may include a medical monitoring device 110 (e.g., a patient monitor), a docking station 120, and one or more intelligent physiological sensors 130a, 130b, 130c, and 130d (collectively referred to as the intelligent physiological sensor 130 herein) in communication. The docking station 120 has processing capability and a fixed location associated with it and provides a hard connection to a network such as the network 140. Moreover, the docking station 120 can determine the type of sensor that is connected via a port on the docking station 120.

A patient 101 may be connected to the intelligent physiological sensor 130 and/or the medical monitoring device 110. Upon admission of a patient to a hospital or other medical environment, the intelligent physiological sensor 130 is paired to the docking station 120, medical monitoring device 110, or the like. At discharge, unpairing of the intelligent physiological sensor 130 from the docking station 120, medical monitoring device 110, or the like. As described further herein, the intelligent physiological sensor 130 may connect to the docking station 120, and the docking station 120 can inform each sensor of its location and each sensor may store and communicate its location on a network. Once the sensor has its location, it can communicate its presence and location to the medical monitoring device 110 as well as other intelligent physiological sensor 130 connected to the docking station 120.

Sensors are devices for measuring one or more patient physiological parameters of the patient 101. This information can, for example, relate to the measured physiological parameters of the patient 101 and the like (e.g., blood pressure, heart related information, pulse oximetry, respiration information, etc.). Thus, each of the intelligent physiological sensor 130 may be a physiological sensor that measures a patient parameter such as blood oxygen, blood pressure, temperature, etc. Each of the intelligent physiological sensor 130 can plug into a port on the docking station 120 or the medical monitoring device 110.

In this application, the term "intelligent physiological sensor" refers to a physiological sensor having the capability to generate a data stream that includes both patient physiological data (data generated from physiological parameter being measured by the sensor) and "sensor parameter data". "Sensor parameter data" is data that is developed by processing the sensor data. For example if the sensor data is ECG then the parameter data is heart rate.

One type of intelligent sensor is based on an intelligent patient front end (IPFE) device. An IPFE device is a physiological measurement device that can provide updated algorithms, features, and software updates for parameter measurement devices without corresponding releases of a new version of host monitor software. IPFEs, together with patient sensors, comprise a physiological patient parameter measurement delivery system.

Another type of intelligent sensor is based on a service-oriented device connectivity (SDC) compliant device. SDC is a web services-based architecture that enables interoperability amongst point-of-care medical devices and data exchange between point-of-care devices and compatible clinical and hospital information systems. SDC enables a hospital's medical technologies to share data and information bi-directionally, securely and dynamically. SDC is more typically used with a sensor that is stationary and is not moved with the patient 101. The docking station 120 can recognize any sensor at any port depending on the implementation. A legacy sensor (i.e., a sensor that is not IPFE) can also be used (e.g., with an appropriate adapter module).

Each intelligent physiological sensor 130 can indicate to the docking station 120 (or the medical monitoring device 110), when plugged into a port on the docking station 120 or the medical monitoring device 110, the type of device it is (e.g., an IPFE device or an SDC compliant device). Each intelligent physiological sensor 130 repeatedly sends a protocol stream (e.g., a data stream) that indicates the type of sensor (along with other data fields) interspersed with the actual patient data of the patient 101. Thus, when plugged into a port on the docking station 120, each sensor provides a protocol stream and the docking station 120 adds location information of the sensor (e.g., that the sensor is at the docking station 120) to the protocol stream as the protocol stream of data is passed on to the medical monitoring station 120 or elsewhere, for example. The location information is not patient data or sensed data. In this manner, a user such as a clinician does not have to manually enter the location data of the sensor(s) into the docking station 120 or the medical monitoring device 110.

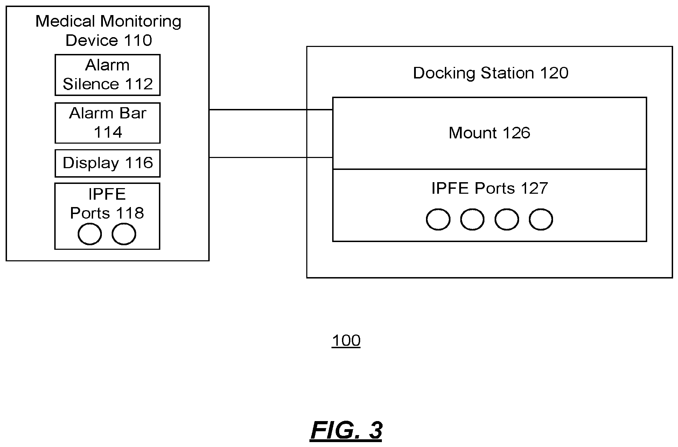

The medical monitoring device 110 is a patient monitor which is used by healthcare facilities to monitor and display information about a patient, such as physiological parameters, vital signs, status of connected devices (e.g., physiological sensors, etc.), and the like. More particularly, the medical monitoring device 110 is configured to acquire and process data generated by at least one physiological sensor configured to monitor a physiological parameter of a patient. The process data generated by sensors such as, without limitation, electrocardiogram (ECG) electrodes, oxygen saturation (SpO2) sensor, blood pressure cuffs, apnea detection sensors, respirators, and others, measure physiological parameters such as blood pressure, heart-related information, pulse oximetry, respiration information, and others. In the healthcare industry, when not being used in transport of a patient or when a patient is ambulatory, monitors can be connected to a monitor mount. Such monitor mounts can provide a variety of functions including providing physical support, a power source, and a conduit to one or more computer networks. Here, the medical monitoring device 110 may be mounted to a mount of the docking station 120 (see e.g., the mount 126 of FIGs. 3-5).

Patient monitors can be portable devices that travel with the patient in order to provide continuous monitoring during care. When a patient arrives at a hospital room or other treatment location, the patient monitor is often plugged into or otherwise connected to a patient monitor mount. Patient monitor mounts provide a physical interface for the patient monitor and are generally fixed at the treatment location. Patient monitor mounts can also provide electrical connection to other devices or infrastructure, such as power to recharge patient monitor batteries, network connectivity to other medical devices or hospital computer systems, and the like.

Patient monitors can rely on the acquisition of physiological signals obtained from front-end analog circuits connected to a patient. Patient monitors can process and/or display analog data derived from the patient. Both patient monitors and patient monitor mounts can include various software applications to process and/or transmit data between each other.

During the course of providing healthcare to patients, practitioners typically connect at least one type of sensor to a patient to sense, derive or otherwise monitor at least one type of patient medical parameter. Such patient connected sensors are further connected to a monitor that includes all relevant electronic components that enable conversion, manipulation and processing of the data sensed by the at least one type of sensor in order to generate patient medical parameters. These patient medical parameters may be stored in one or more modules and are usable by healthcare practitioners (e.g., nurses, doctors, physician assistants, or any other person charged with providing a healthcare service to a patient) in monitoring a patient and determining a course of healthcare to be provided to the patient. Additionally or alternatively, the one or more modules may contain data, such as patient treatment data, to be transferred to the monitor and/or a dock.

As described further herein, the medical monitoring device 110 is detachably secure (i.e., removably connectable) with, or otherwise can physically interface with, a mount of the docking station 120, and the intelligent physiological sensor 130 may be detachably securely connected to ports on the docking station 120.

The network 140 may be a variety of network types including the public switched telephone network (PSTN), a cellular telephone network, and a packet switched network (e.g., the Internet). Although only one medical monitoring device 110, one docking station 120, and four intelligent physiological sensor 130 are shown in FIG. 1, there is no limit to the number of medical monitoring devices 110, docking stations 120, and intelligent physiological sensor 130 that may be supported.

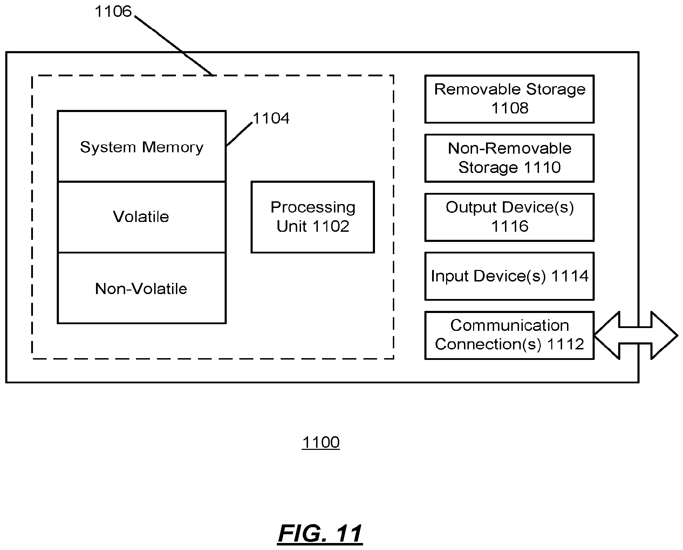

In some implementations, the docking station 120 may be connected to a server 150 and/or a patient database 152, e.g., via a network such as the network 140. The server 150 may perform processing of data received from the docking station 120 and send data (e.g., results, patient information, instructions, location, etc.) to the docking station 120 for display, further processing, or to provide to the medical monitoring device 110 or one or more of the intelligent physiological sensor 130. The patient database 152 may store data for the patient 101, such as data received from the intelligent physiological sensor 130 or results and other information from processing performed by the medical monitoring device 110, the docking station 120, the intelligent physiological sensor 130, the server 150, or other computing devices. The medical monitoring device 110, the docking station 120, and the server 150 may be implemented using (or comprise or be integral with) a variety of computing devices such as desktop computers, laptop computers, and tablets. Other types of computing devices may be supported. A suitable computing device is illustrated in FIG. 11 as the computing device 1100.

In some implementations, the medical monitoring device 110 may comprise a personal computer (PC) that is attached via a network to the docking station 120. The medical monitoring device 110 may be connected to, or comprise, a core system which is a software package on the network. The core system aggregates the sensed data and other information pertaining to the patient (i.e., patient information) and is configured to display the patient information on the medical monitoring device 110 and/or the docking station 120 depending on the embodiment.

The docking station 120 is used to mount the medical monitoring device 110 and connect the medical monitoring device 110 to the network 140 (in this example, an SDC network) as well as the intelligent physiological sensor 130. The IPFE devices conform to the structure and rules of the SDC network. The SDC network provides means for coupling devices (e.g., sensors) through the use of location contexts and patient contexts. The location context is populated into a sensor (e.g., into an IPFE device or an SDC device) that is connected to the docking station 120.

Depending on the implementation, the docking station 120 has ports for IPFE devices as well as ports for SDC devices which may include SDC Ethernet devices. When a sensor is plugged into the IPFE port or the SDC port, the processor in the docking station 120 connects to the sensor and populates the SDC location context field in the sensor's medical database. The sensor's medical database can include data objects that are provided by a particular medical device including descriptive and state information. The sensor's medical database can include various descriptive and state elements, which may be used for subsequent processing. Additionally, the sensor's medical database can include information on how to use the data produced by the sensor, how data is formatted, how often the data is updated, how the data should be displayed, and how the data should be labelled. Additionally, because the docking station 120 is a communication hub for SDC and IPFE device sensors, it is advantageous to have a user interface for status of the sensors. Accordingly, the docking station 120 may have a user interface to allow a clinician to see the status of the connected sensors.

This topology allows sensors to be connected to the SDC network without having to be connected to the medical monitoring device 110. Thus, functionality is provided without the medical monitoring device 110 being plugged into the docking station 120, in some embodiments. For example, the intelligent physiological sensor 130 can be tested without the medical monitoring device 110 being attached to the docking station 120 or the intelligent physiological sensor 130. Additionally, the firmware of the sensors can be updated, a sensor calibration can be performed, and/or the sensors can be warmed up without the medical monitoring device 110 being attached to the docking station 120 or the intelligent physiological sensor 130.

In addition, the Ethernet connection of SDC compliant devices can be intercepted and their location context can be added to their database. Once the location context is loaded, the docking station 120 terminates its local connection to the sensor and connects the sensor to its internal Ethernet hub for access to the network.

An advantage of having docking station ports that all associate with one location is that it can save time compared to having to manually configure each device to be associated with a patient. Medical equipment such as sensors are added to and removed from a patient as the patient's condition changes. Sometimes equipment needs to be added quickly to intervene or monitor a change in condition. Thus, the location context is configured for a sensor by plugging all the sensors into a single physical hub that is near the patient and is already used to concentrate the connections of a medical monitoring device.

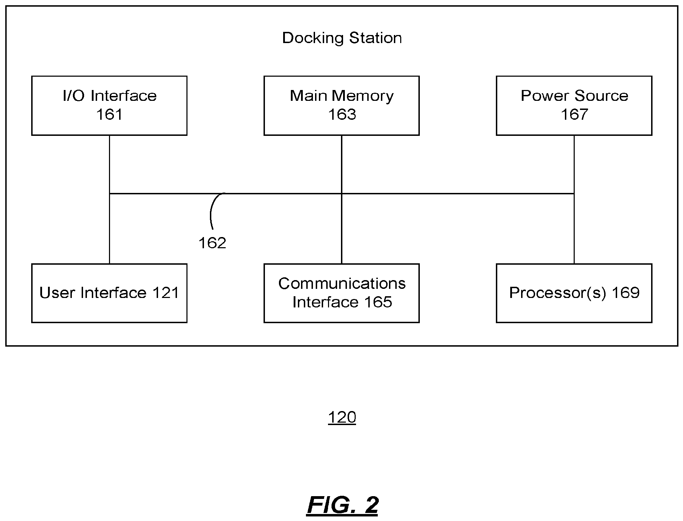

FIG. 2 shows a schematic diagram of a docking station 120 in accordance with described embodiments. It is contemplated by the present disclosure that the docking station 120 includes electronic components or electronic computing devices operable to receive, transmit, process, store, and/or manage data and information associated with the systems and methods described further herein, which encompasses any suitable processing device adapted to perform computing tasks consistent with the execution of computer-readable instructions stored in memory or computer-readable recording medium.

Referring to FIG. 2, the example docking station 120 includes one or more memories or memory locations including a main memory 163 as well as an I/O interface 161, a user interface 121, a communications interface 165, one or more processors 169, and an optional power source 167. The main memory 163 can be a random access memory (RAM), a memory buffer, a hard drive, a database, an erasable programmable read only memory (EPROM), an electrically erasable programmable read only memory (EEPROM), a read only memory (ROM), a flash memory, hard disk or any other various layers of memory hierarchy.

The main memory 163 can be used to store any type of instructions associated with algorithms, processes, or operations for controlling the general functions of the docking station 120 including the operations described further herein as well as any operating system such as Linux, UNIX, Windows Server, or other customized and proprietary operating systems.

The optional power source 167 can be used to power the various components of the docking station 120. The power source 167 can include a self-contained power source such as a battery pack and/or include an interface to be powered through an electrical outlet (either directly or by way of the docking station 120 or the medical monitoring device 110 or a device such as a sensor 130). The power source 167 can also be a rechargeable battery that can be detached allowing for replacement. In certain embodiments, the docking station 120 can only be powered and render information when secured or otherwise connected to one or more of a device such as a sensor 130 and a device mount.

The I/O interface 161 can be an interface for enabling the transfer of information between the docking station 120 and the external devices such as a sensor 130 or the medical monitoring device 110 that need special communication links for interfacing with the one or more processors 169. The I/O interface 161 can be implemented to accommodate various connections to the docking station 120 that include, but is not limited to, a universal serial bus (USB) connection, a parallel connection, a serial connection, an Ethernet connection, coaxial connection, a wireless connection, a High-Definition Multimedia Interface (HDMI) connection or other similar connection known in the art for connecting to external devices.

The docking station 120 can provide the medical monitoring device 110 with information about what data is being provided and the properties for various sub-systems as well as enable the display of elements within the medical monitoring device 110.

The user interface 121 is implemented for allowing communication between a user and the docking station 120. The user interface 121 includes, but is not limited to, a liquid crystal display (LCD), cathode ray tube (CRT), thin film transistor (TFT), light-emitting diode (LED), high definition (HD) or other similar display device with touch screen capabilities. Other kinds of input devices can be used to provide for interaction with a user as well. For example, feedback provided to the user can be any form of sensory feedback (e.g., visual feedback, auditory feedback by way of a speaker, or tactile feedback), and input from the user can be received in any form, including acoustic, speech, or tactile input. Input devices and a microphone can be coupled to and convey information via the internal bus 162 by way of an input device interface. A speaker can be coupled to and receive information via the internal bus 162 by way of a speaker interface. The docking station 120 can omit one or more of the display, display interface, input device, microphone, speaker, input device interface, and speaker interface.

The communications interface 165 is a software and/or hardware interface implemented to establish a connection between the docking station 120 and the server 150 in the system described in FIG. 1. It is contemplated by the present disclosure that the communications interface 165 includes software and/or hardware interface circuitry for establishing communication connections with the rest of the system using both wired and wireless connections for establishing connections to, for example, a local area networks (LANs), wide area networks (WANs), metropolitan area networks (MANs) personal area networks (PANs), wireless local area networks (WLANs), system area networks (SANs), and combinations thereof.

The communications interface 165 allows the docking station 120 to communicate with one or more computing networks and devices (e.g., the medical monitoring device 110). The communications interface 165 can include various network cards, interfaces or circuitry to enable wired and wireless communications with such computing networks and devices. The communications interface 165 can also be used to implement, for example, a Bluetooth connection, a cellular network connection, and a Wi-Fi connection. Other wireless communication connections implemented using the communications interface 165 include wireless connections that operate in accordance with, but are not limited to, IEEE 802.11 protocol, a Radio Frequency For Consumer Electronics (RF4CE) protocol, ZigBee protocol, and/or IEEE 802.15.4 protocol.

The communications interface 165 can also enable direct (i.e., device-to-device) communications (e.g., messaging, signal exchange, etc.) such as from the docking station 120 to the medical monitoring device 110 using, for example, a USB connection, coaxial connection, or other similar electrical connection. The communications interface 165 can enable direct (i.e., device-to-device) to other device such as to a tablet, PC, or similar electronic device; or to an external storage device or memory.

The one or more processors 169 are used for controlling the general operations of the docking station 120. Each one of the one or more processors 169 can be, but are not limited to, a central processing unit (CPU), a hardware microprocessor, a multicore processor, a single core processor, a field programmable gate array (FPGA), an application specific integrated circuit (ASIC), a digital signal processor (DSP), or other similar processing device capable of executing any type of instructions, algorithms, or software for controlling the operation of the docking station 120. Communication between the components of the docking station 120 (e.g., the user interface 121, the I/O interface 161, the main memory 163, the communications interface 165, the power source 167, and the processor(s) 169) are established using an internal bus 162.

FIG. 3 shows a schematic diagram of aspects of the environment 100 of FIG. 1 with additional detail, in particular the medical monitoring device 110 and the docking station 120.

The medical monitoring device 110 comprises an alarm silence 112, an alarm bar 114, a display 116, and IPFE ports 118. The alarm silence 112 can be actuated or otherwise activated to silence an alarm or other alert generated or indicated by the medical monitoring device 110. The alarm bar 114 is configured and equipped to provide visual and/or audible indications of alarm conditions. The display 116 is configured to display information pertaining to the patient 101 and other information, e.g., to a user such as a clinical or other health care provider. The IPFE ports 118 are configured to receive IPFE sensors which may plug into the IPFE ports 118.

A medical monitoring device, such as the medical monitoring device 110 can include a sensor interface (shown as the IPFE ports 118) that can be used to connect via wired and/or wireless interfaces to one or more physiological sensors (e.g., the intelligent physiological sensor 130) and/or medical devices (e.g., ECG electrodes, SPO2 sensors, blood pressure cuffs, apnea detection sensors, respirators, etc.) associated with the patient 101. The medical monitoring device 110 can include one or more processors (e.g., programmable data processors, etc.) which can execute various instructions stored in memory of the monitor the medical monitoring device. Various data and graphical user interfaces can be conveyed to a user via an electronic visual display, such as the display 116. This information can, for example, relate to the measured physiological parameters of the patient 101 and the like (e.g., blood pressure, heart related information, pulse oximetry, respiration information, etc.). Other types of information can also be conveyed by the electronic visual display 116. In some variations, the electronic visual display 116 includes a touch screen interface.

The medical monitoring device 110 can additionally include a communications interface (not shown) which allows the medical monitoring device directly or indirectly (via, for example, the docking station 120) to access one or more computing networks. The communications interface can include various network cards/interfaces to enable wired and wireless communications with such computing networks. The communications interface can also enable direct (i.e., device-to-device, etc.) communications (i.e., messaging, signal exchange, etc.) such as from the docking station 120 to the medical monitoring device 110.

The medical monitoring device 110 can optionally also include a power source and/or conduit (not shown) that can be used to power the various components of the medical monitoring device 110. The power source/conduit can include a self-contained power source such as a battery pack and/or the power source/conduit can include an interface to be powered through an electrical outlet (either directly or by way of the docking station 120).

The docking station 120 comprises a mount 126 and IPFE ports 127. The mount 126 is configured to receive, or otherwise connect to, the medical monitoring device 110. It is contemplated that the medical monitoring device 110 and the docking station 120 are detachably secure (i.e., removably connectable). In some implementations, the medical monitoring device 110 and the docking station 120 may form an integral unit. The IPFE ports 127 are configured to receive IPFE sensors which may plug into the IPFE ports 127.

The mount 126 can be an optional component in some implementations. The mount 126 can provide physical support, a power source, and a conduit to one or more computer networks. In certain embodiments, when the medical monitoring device 110 is not used in transport of a patient, the mount 126 can be connected. It is contemplated by the present disclosure that, in certain embodiments, the medical monitoring device 110 can connect to the mount 126. In some embodiments, communication from a sensor to the medical monitoring device 110 is established through the mount 126.

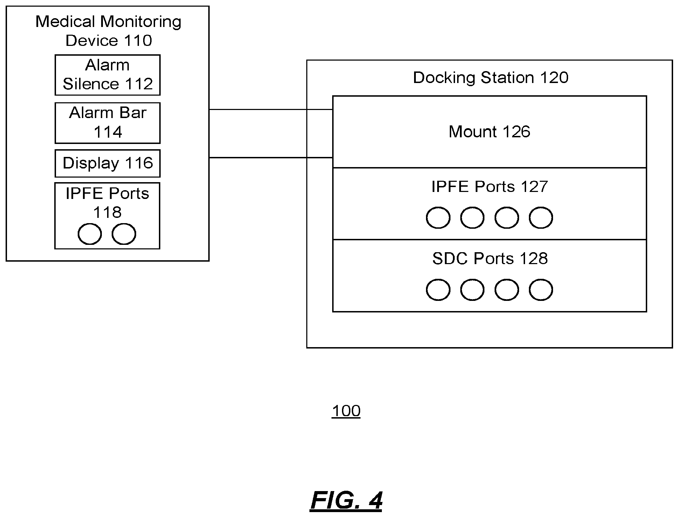

FIG. 4 shows a schematic diagram of aspects of the environment 100 of FIG. 1 with additional detail, in particular the medical monitoring device 110 and the docking station 120. Similar to FIG. 3, the medical monitoring device 110 comprises an alarm silence 112, an alarm bar 114, a display 116, and IPFE ports 118. The medical monitoring device 110 is similar to that described with respect to FIG. 3 and therefore its further description with respect to FIG. 4 is omitted here for brevity.

Similar to FIG. 3, the docking station 120 comprises a mount 126 and IPFE ports 127. The mount 126 and the IPFE ports 127 are described with respect to FIG. 3 and therefore their descriptions with respect to FIG. 4 are omitted for brevity. In the embodiment of FIG. 4, the docking station further comprises SDC ports 128. The SDC ports 128 are configured to receive SDC sensors which may plug into the SDC ports 128.

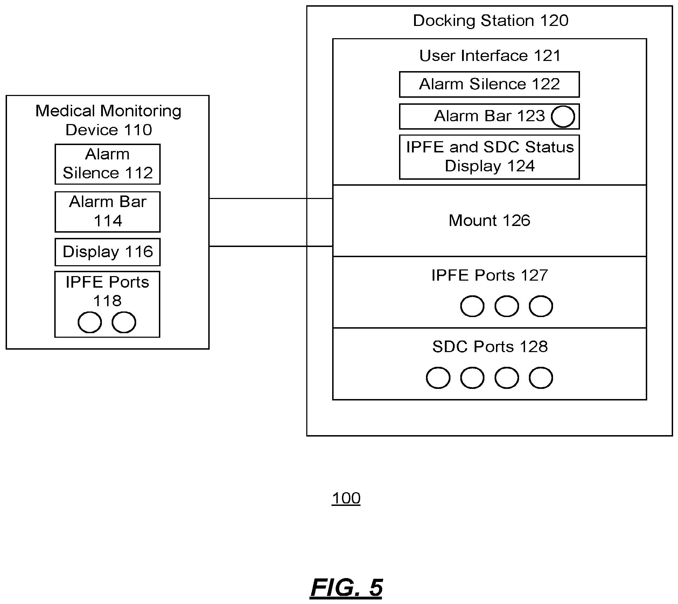

FIG. 5 shows a schematic diagram of aspects of the environment 100 of FIG. 1 with additional detail, in particular the medical monitoring device 110 and the docking station 120. Similar to FIG. 3, the medical monitoring device 110 comprises an alarm silence 112, an alarm bar 114, a display 116, and IPFE ports 118. The medical monitoring device 110 is similar to that described with respect to FIG. 3 and therefore its further description with respect to FIG. 5 is omitted here for brevity.

The docking station 120 comprises a mount 126, IPFE ports 127, and SDC ports 128. The mount 126 and the IPFE ports 127 are described with respect to FIG. 3, and the SDC ports 128 are described with respect to FIG. 4 and therefore their descriptions with respect to FIG. 5 are omitted for brevity. In the embodiment of FIG. 5, the docking station 120 further comprises a user interface 121.

The user interface 121 comprises an alarm silence 122, an alarm bar 123, and an IPFE and SDC status display 124. The alarm silence 122 can be actuated or otherwise activated to silence an alarm or other alert generated or indicated by the docking station 120. The alarm bar 123 is configured and equipped to provide visual and/or audible indications of alarm conditions. The alarm bar 123 may comprise a speaker.

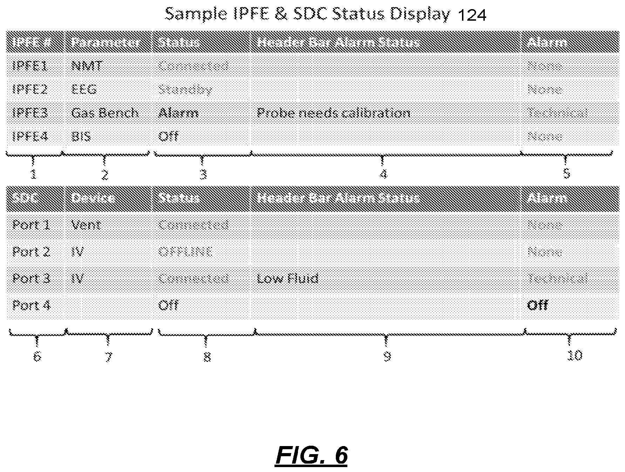

The IPFE and SDC status display 124 is a display of the status of the sensors plugged into the IPFE ports 127 and the SDC ports 128. The display 124 informs the clinician the overall status of the intelligent physiological sensor 130 connected to the docking station 120. An example of the IPFE and SDC status display 124 is described with respect to FIG. 6.

The display could include display fields as follows: identifies IPFE connector on docking station; identifies parameter plugged into IPFE port; identifies IPFE status including discharge; displays alarm bar header; alarm state, identifies reason for alarm tone and flashing header bar; identifies SDC Ethernet connector; identifies SDC device plugged into port; identifies Ethernet connection status; identifies distributed alarm system (DAS) status, and may be synchronized with the alarm bar 123 (e.g., a local speaker and flashing header).

FIG. 6 shows an example of the IPFE and SDC status display 124 on a docking station in accordance with described embodiments. The status display 124 may be rendered or otherwise displayed as the IPFE and SDC status display on the user interface 121 of the docking station 120 in some implementations.

The IPFE sensor number is shown in column 1. The parameter that is being sensed is shown in column 2. The status of the sensor is shown in column 3. The header bar alarm status is shown in column 4. The alarm status is shown in column 5. If the alarm status is "active", the type of alarm is preferably displayed (e.g., "technical" as shown in connection with sensor IPFE3).

The SDC sensor port is shown in column 6. The sensor device type is shown in column 7. The status of the sensor is shown in column 8. The header bar alarm status is shown in column 9. The alarm status is shown in column 10.

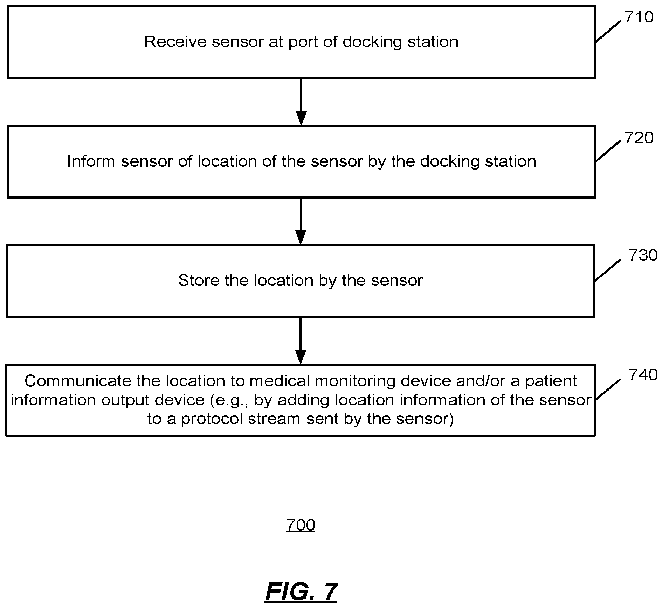

FIG. 7 is an operational flow of an implementation of a method 700 for providing location context with a docking station in accordance with described embodiments. The method 700 may be implemented using the docking station 120 in conjunction with the environment 100, in some implementations.

At 710, a sensor is received at a port of a docking station, such as the docking station 120. For example, an IPFE sensor is plugged into an IPFE port 127 or an SDC sensor is plugged into an SDC port 128 of the docking station 120.

At 720, the docking station 120 informs the sensor of the location of the sensor.

At 730, the sensor stores the location.

At 740, the location is communicated to a medical monitoring device and/or a patient information output device that aggregates or displays patient information. The location may be communicated by adding location information of the sensor to a protocol stream sent by the sensor.

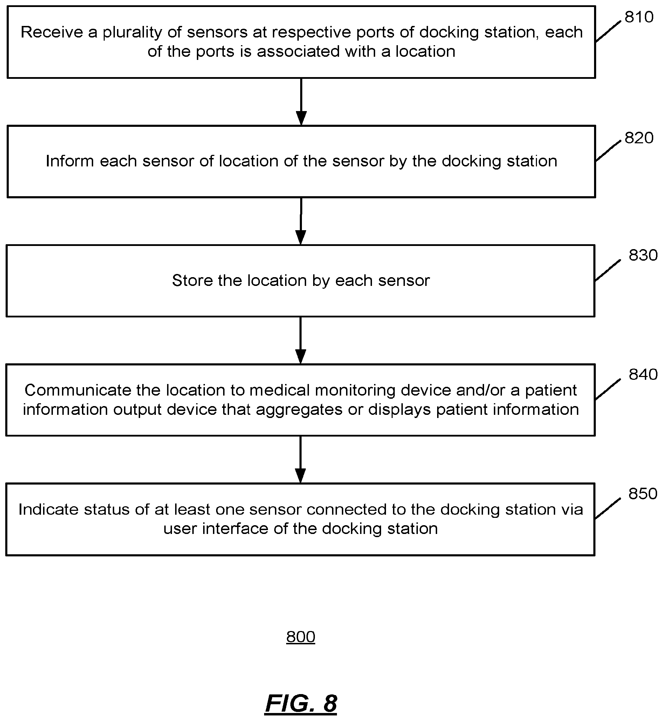

FIG. 8 is an operational flow of an implementation of a method 800 for providing location context with a docking station in accordance with described embodiments. The method 800 may be implemented using the docking station 120 in conjunction with the environment 100, in some implementations.

At 810, multiple sensors, such as one or more IPFE sensors and/or one or more SDC sensors, are received at respective ports of a docking station, such as the IPFE ports 127 and/or the SDC ports 128 of the docking station 120. Each of the ports is associated with a location. In some implementations, the location is the location of the docking station, so that each of the sensors that are received at the ports will have the same location.

At 820, the docking station 120 informs each of the sensors of the location of that sensor.

At 830, each sensor stores its location.

At 840, the location is communicated to a medical monitoring device and/or a patient information output device that aggregates or displays patient information.

At 850, the status of at least one of the sensors that is connected to the docking station is indicated on a user interface of the docking station. For example, the user interface 121 of the docking station 120 generate and show a display such as the IPFE and SDC status display 124.

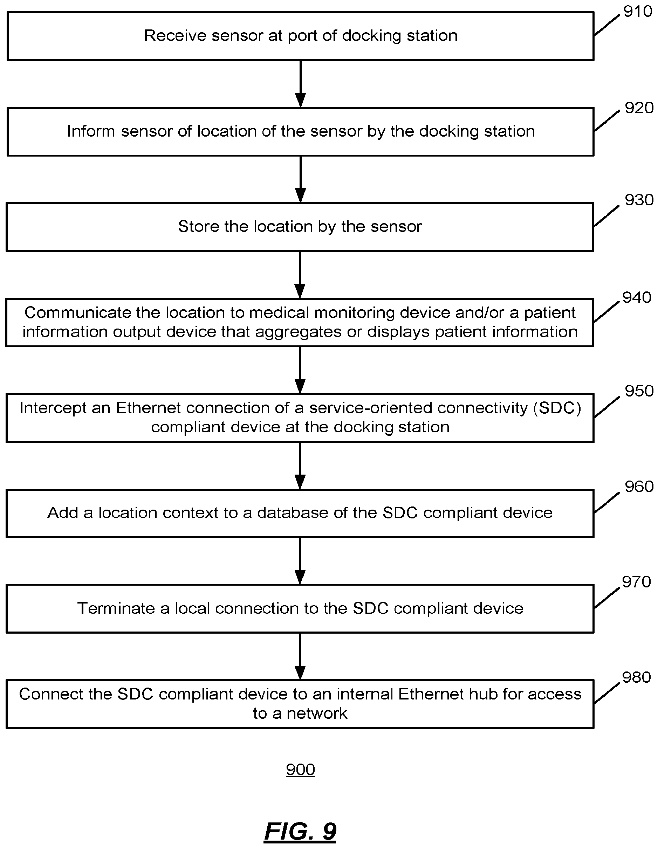

FIG. 9 is an operational flow of an implementation of a method 900 for providing location context with a docking station in accordance with described embodiments. The method 900 may be implemented using the docking station 120 in conjunction with the environment 100, in some implementations.

At 910, similar to 710, a sensor is received at a port of a docking station, such as the docking station 120.

At 920, similar to 720, the docking station 120 informs the sensor of the location of the sensor.

At 930, similar to 730, the sensor stores the location.

At 940, similar to 740, the location is communicated to a medical monitoring device and/or a patient information output device that aggregates or displays patient information.

At 950, an Ethernet connection of a SDC compliant device is intercepted at the docking station.

At 960, a location context is added to a database of the SDC compliant device.

At 970, a local connection to the SDC compliant device is terminated.

At 980, the SDC compliant device is connected to an internal Ethernet hub for access to a network.

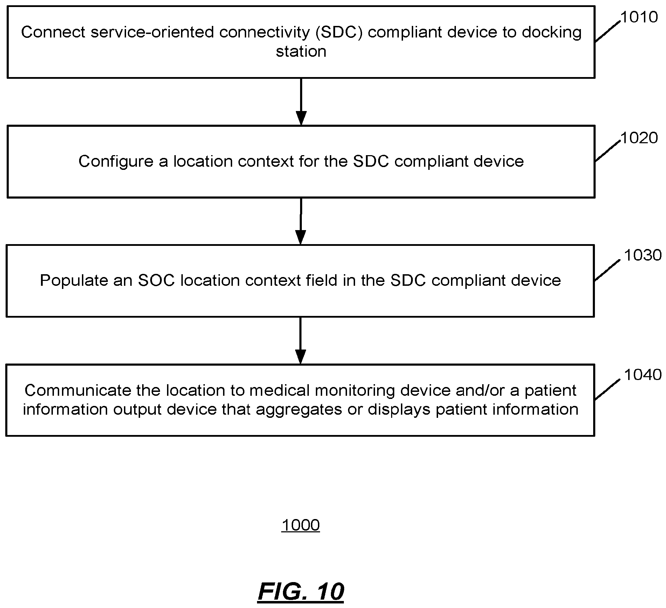

FIG. 10 is an operational flow of an implementation of a method 1000 for providing location context with a docking station in accordance with described embodiments. The method 1000 may be implemented using the docking station 120 in conjunction with the environment 100, in some implementations.

At 1010, an SDC compliant device is connected to a docking station, such as the docking station 120.

At 1020, a location context is configured for the SDC compliant device.

At 1030, an SDC location context field is populated in the SDC compliant device.

At 1040, the location is communicated to a medical monitoring device and/or a patient information output device that aggregates or displays patient information.

Implementations may include some or all of the following features. A docking station comprises a power source, a processor, a mount configured to removably receive a multi-parameter patient monitor, a plurality of sensor ports configured to mechanically and electrically connect to physiological sensors, a communications interface configured to communicate with an SDC network, and an I/O interface configured to communicate with each physiological sensor connected to one of the plurality of sensor ports and the multi-parameter patient monitor connected to the mount. The docking station is associated with a first location on the SDC network. Through the I/O interface, a sensor data signal is received from a physiological sensor that is SDC compliant or IPFE compliant. The sensor data signal in the docking station is appended to include a location parameter that associates the physiological sensor with the first location. The appended sensor data from the docking station is transmitted through the communications interface to at least one selected from the group of the multi-parameter patient monitor connected to the mount and the SDC network. The appended sensor data is used to associate the physiological sensor with the first location on the SDC network.

In an implementation, when the physiological sensor is SDC compliant, the appended sensor data from the docking station is transmitted through the communications interface to the SDC network. Alternatively or additionally, the communications interface and the SDC network can be used for other equipment such as a ventilator and/or an IV pump, for example. Such equipment needs to be associated with a patient and plugging this equipment into the docking station provides an automatic way to associate the equipment with the patient.

In an implementation, when the physiological sensor is IPFE compliant, the appended sensor data from the docking station is transmitted through the communications interface to the multi-parameter patient monitor connected to the mount.

FIG. 11 shows an exemplary computing environment in which example embodiments and aspects may be implemented. The computing device environment is only one example of a suitable computing environment and is not intended to suggest any limitation as to the scope of use or functionality.

Numerous other general purpose or special purpose computing devices environments or configurations may be used. Examples of well-known computing devices, environments, and/or configurations that may be suitable for use include, but are not limited to, personal computers, server computers, handheld or laptop devices, multiprocessor systems, microprocessor-based systems, network personal computers (PCs), minicomputers, mainframe computers, embedded systems, distributed computing environments that include any of the above systems or devices, and the like.

Computer-executable instructions, such as program modules, being executed by a computer may be used. Generally, program modules include routines, programs, objects, components, data structures, etc. that perform particular tasks or implement particular abstract data types. Distributed computing environments may be used where tasks are performed by remote processing devices that are linked through a communications network or other data transmission medium. In a distributed computing environment, program modules and other data may be located in both local and remote computer storage media including memory storage devices.

With reference to FIG. 11, an exemplary system for implementing aspects described herein includes a computing device, such as computing device 1100. In its most basic configuration, computing device 1100 typically includes at least one processing unit 1102 and memory 1104. Depending on the exact configuration and type of computing device, memory 1104 may be volatile (such as random access memory (RAM)), non-volatile (such as read-only memory (ROM), flash memory, etc.), or some combination of the two. This most basic configuration is illustrated in FIG. 11 by dashed line 1106.

Computing device 1100 may have additional features/functionality. For example, computing device 1100 may include additional storage (removable and/or non-removable) including, but not limited to, magnetic or optical disks or tape. Such additional storage is illustrated in FIG. 11 by removable storage 1108 and non-removable storage 1110.

Computing device 1100 typically includes a variety of computer readable media. Computer readable media can be any available media that can be accessed by the device 1100 and includes both volatile and non-volatile media, removable and non-removable media.

Computer storage media include volatile and non-volatile, and removable and non-removable media implemented in any method or technology for storage of information such as computer readable instructions, data structures, program modules or other data. Memory 1104, removable storage 1108, and non-removable storage 1110 are all examples of computer storage media. Computer storage media include, but are not limited to, RAM, ROM, electrically erasable program read-only memory (EEPROM), flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by computing device 1100. Any such computer storage media may be part of computing device 1100.

Computing device 1100 may contain communication connection(s) 1112 that allow the device to communicate with other devices. Computing device 1100 may also have input device(s) 1114 such as a keyboard, mouse, pen, voice input device, touch input device, etc. Output device(s) 1116 such as a display, speakers, printer, etc. may also be included. All these devices are well known in the art and need not be discussed at length here.

Although exemplary implementations may refer to utilizing aspects of the presently disclosed subject matter in the context of one or more stand-alone computer systems, the subject matter is not so limited, but rather may be implemented in connection with any computing environment, such as a network or distributed computing environment. Still further, aspects of the presently disclosed subject matter may be implemented in or across a plurality of processing chips or devices, and storage may similarly be effected across a plurality of devices. Such devices might include personal computers, network servers, and handheld devices, for example.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

It will be further understood that various changes in the details, materials, and arrangements of the parts that have been described and illustrated in order to explain the nature of the described embodiments might be made by those skilled in the art without departing from the scope expressed in the following claims.

## Claims

1. A method comprising:
a. providing a first docking station having a power source, a processor, a mount configured to removably receive a multi-parameter patient monitor, at least one sensor port configured to mechanically and electrically connect to an intelligent physiological sensor, a docking station communications interface configured to communicate with a hospital network, and a docking station I/O interface configured to communicate with the intelligent physiological sensor connected to each of the at least one sensor port and the multi-parameter patient monitor connected to the mount, the hospital network being adapted to store location data for the first docking station, the intelligent physiological sensor being adapted to generate a sensor data signal that includes sensor parameter data;
b. associating the first docking station with a first location on the hospital network;
c. establishing an electrical connection between the docking station I/O interface and a sensor data signal for the intelligent physiological sensor when the intelligent physiological sensor is connected to the at least one sensor port; and
d. appending the sensor parameter data using the docking station I/O interface to include a sensor location parameter that associates the intelligent physiological sensor with the first location.

2. The method of claim 1, further comprising:
e. transmitting the appended sensor parameter data from the first docking station through the communications interface to at least one selected from the group of the multi-parameter patient monitor connected to the mount and the hospital network; and
f. using the appended sensor data to associate the intelligent physiological sensor with the first location on the hospital network,
optionally wherein step e comprises transmitting the appended sensor parameter data from the first docking station through the communications interface to the hospital network, and further optionally wherein the first docking station is adapted to perform step e regardless of whether the multi-parameter patient monitor is connected to the mount.

3. The method of any one of claims 1 or 2, further comprising:
g. terminating the connection between the docking station I/O interface after steps c and d have been completed.

4. The method of any one of the preceding claims, wherein the intelligent physiological sensor is one selected from the group of an intelligent patient front end (IPFE) sensor and a service-oriented device connectivity (SDC) compliant sensor.

5. The method of any one of the preceding claims, wherein the communication with the hospital network is facilitated by one or more network ports on the first docking station.

6. The method of any one of the preceding claims, wherein the first docking station further comprises a graphical user interface and the method further comprises:
h. displaying on the graphical user interface status information for each intelligent physiological sensor electrically connected to the at least one sensor port, optionally wherein the status information comprises at least one selected from the group of a sensor type, a sensor status, a status description, and an alarm status.

7. The method of any one of the preceding claims, further comprising:
i. generating at least one selected from the group of an audio alarm and a visual alarm through the graphical user interface when the alarm status of at least one of the intelligent physiological sensors electrically connected to the at least one sensor port is active, optionally further comprising:
j. enabling a user to silence the alarm generated by step i using the graphical user interface.

8. The method of any one of the preceding claims, further comprising:
k. calibrating at least one of the intelligent physiological sensors;
wherein the first docking station is adapted to perform step (k) even if the multi-parameter patient monitor is not connected to the mount, and/or
l. warming at least one of the intelligent physiological sensors;
wherein the first docking station is adapted to perform step (I) even if the multi-parameter patient monitor is not connected to the mount.

9. The method of any one of the preceding claims, further comprising:
m. disconnecting the intelligent physiological sensor from the first docking station;
n. automatically associating the intelligent physiological sensor with the first location when step (m) is performed;
o. mechanically and electrically connecting the intelligent physiological sensor to a second docking station, the second docking station being associated with a second location on the hospital network; and
p. automatically associating the intelligent physiological sensor with the second location when step (o) is performed.

10. A docking station comprising:
a power source;
a processor;
a mount configured to removably receive a multi-parameter patient monitor;
at least one sensor port configured to mechanically and electrically connect to an intelligent physiological sensor;
a docking station communications interface configured to communicate with a hospital network and to associate a first location with the docking station; and
a docking station I/O interface configured to communicate with the intelligent physiological sensor connected to each of the at least one sensor port and the multi-parameter patient monitor connected to the mount,
wherein the docking station I/O interface is further configured to append the first location to a sensor data signal of the intelligent physiological sensor when the intelligent physiological sensor is electrically connected to the at least one sensor port.

11. The docking station of claim 10, wherein the docking station communications interface is adapted to transmit the sensor data signal to the hospital network even if the multi-parameter patient monitor is not received in the mount.

12. The docking station of any one of claims 10 or 11, wherein the docking station I/O interface is configured to terminate a connection with the intelligent physiological sensor after the first location is appended to the sensor data signal.

13. The docking station of any one of claims 10-12, wherein the at least one sensor port comprises at least one intelligent patient front end (IPFE) port and/or at least one service-oriented device connectivity (SDC) port.

14. The docking station of any one of claims 10-13, further comprising a graphical user interface adapted to provide status information for each intelligent physiological sensor electrically connected to the at least one sensor port, optionally wherein the status information comprises at least one selected from the group of a sensor type, a sensor status, a status description, and an alarm status.

15. The docking station of any one of claims 10-14, wherein the graphical user interface is adapted to generate at least one selected from the group of an audio alarm and a visual alarm through the graphical user interface when the alarm status of at least one of the intelligent physiological sensors electrically connected to the at least one sensor port is active, and optionally wherein the graphical user interface is adapted to enable a user to silence the alarm generated by the graphical user interface.
